(19) Europäisches Patentamt
European Patent Office
Office européen des brevets

(11) EP 0 471 809 B1

(12) # EUROPÄISCHE PATENTSCHRIFT

(45) Veröffentlichungstag und Bekanntmachung des Hinweises auf die Patenterteilung:
**29.05.1996 Patentblatt 1996/22**

(21) Anmeldenummer: **91904470.1**

(22) Anmeldetag: **22.02.1991**

(51) Int Cl.$^6$: **C07C 229/24**, C07C 227/18, C07K 5/06

(86) Internationale Anmeldenummer:
**PCT/EP91/00332**

(87) Internationale Veröffentlichungsnummer:
**WO 91/13861 (19.09.1991 Gazette 1991/22)**

(54) **VERFAHREN ZUR HERSTELLUNG VON N-GESCHÜTZTEN (2S,3S)-3-ETHYLASPARAGINSÄUREDIALKYLESTERN**

METHOD FOR PREPARING N-PROTECTED (2S,3S)-3-ETHYLASPARTIC ACID DIALKYL ESTERS

PROCEDE DE PRODUCTION DE DIALKYLESTERS D'ACIDE (2S,3S)-3-ETHYLASPARTIQUE PROTEGES PAR N

(84) Benannte Vertragsstaaten:
**AT BE CH DE ES FR GB IT LI NL SE**

(30) Priorität: **07.03.1990 DE 4007038**

(43) Veröffentlichungstag der Anmeldung:
**26.02.1992 Patentblatt 1992/09**

(73) Patentinhaber: **MERCK PATENT GmbH**
**D-64271 Darmstadt (DE)**

(72) Erfinder:
- **HEYWANG, Ulrich**
  **D-6100 Darmstadt 14 (DE)**
- **SCHWARTZ, Harry**
  **D-6238 Hofheim-Diedenbergen (DE)**
- **CASUTT, Michael**
  **D-6106 Erzhausen (DE)**

(56) Entgegenhaltungen:
- **THE JOURNAL OF ORGANIC CHEMISTRY, Band 54, Nr. 13, veröffentlicht 1989, J.P. WOLF et al. "Conformationally constrained peptides.**
- **TETRAHEDRON, Band 43, Nr. 21, veröffentlicht 1987, Oxford M. AKHTAR et al. "Enatiospecific synthesis of 3-substituted aspartic**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 471 809 B1

## Beschreibung

Die Erfindung betrifft ein Verfahren zur Herstellung von N-geschützten (2S,3S)-3-Ethylasparaginsäuredialkylestern bzw. deren Salze, aus ungereinigter, durch enzymatische Aminierung von Ethylfumarsäure erhältlicher (2S,3S)-3-Ethylasparaginsäure.

N-geschützte (2S,3S)-3-Ethylasparaginsäuredialkylester sind wertvolle Zwischenprodukte zur Herstellung eines γ-Lactam-verbrückten 2S,3R,2'S-Isoleucin-Alanin-Dipeptides (z.B. J.P. Wolf, H. Rapoport, J. Org. Chem. 1989, 54, 3164-3173).

Vor- und nachstehend bedeuten:

PhFl 9-Phenyl-fluorenyl
E $-CO_2CH_3$

Der Dimethylester I läßt sich nach J.P. Wolf und H.Rapoport (loc. cit.) durch enantioselektive Alkylierung von N-9-Phenylfluorenylasparaginsäuredimethylester gemäß Schema 1 herstellen.

Schema 1

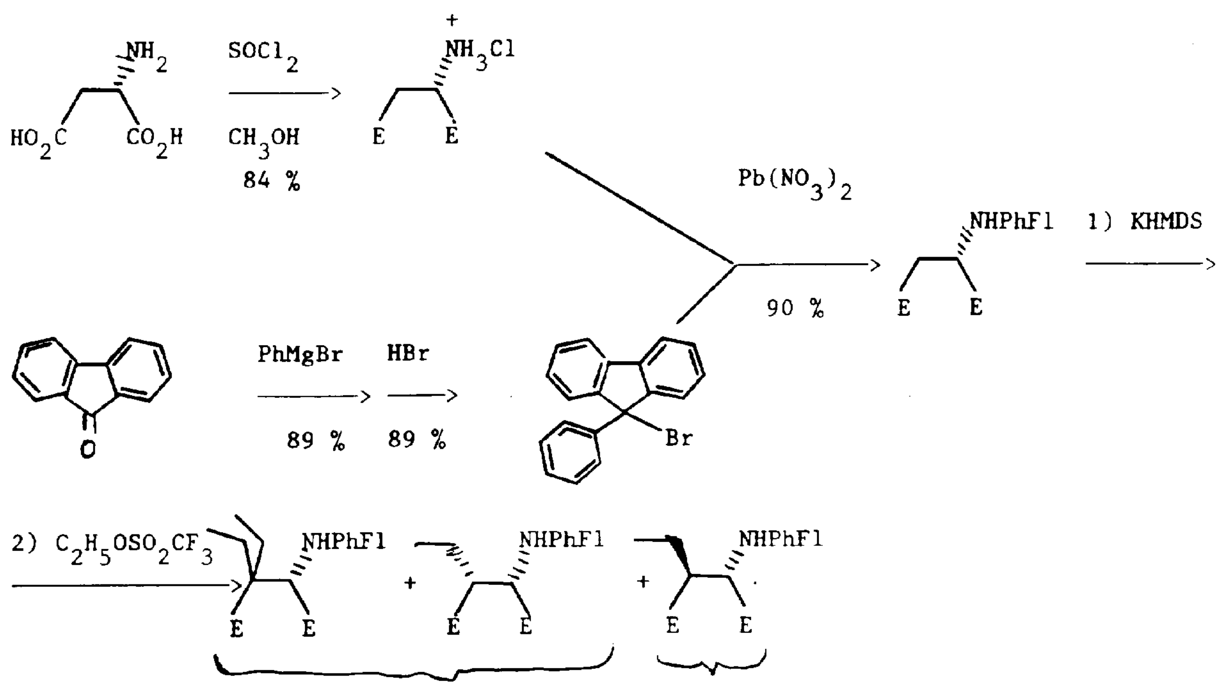


abtrennbare Nebenprodukte I

KHMDS = Kaliumhexamethyldisilazid

Dieses Verfahren weist jedoch mehrere Nachteile auf, die eine Einführung dieses Syntheseweges in die großtechnische Produktion unmöglich machen. Einerseits ist die sperrige Aminoschutzgruppe 9-Phenylfluorenyl, die für das Gelingen der enantioselektiven Alkylierung unumgänglich ist, schwer zugänglich und schlecht einführbar, andererseits ist die Base Kaliumhexamethyldisilazan, die durch Umsetzung von Kaliumhydrid mit Di(trimethylsilyl)amin hergestellt wird, nur unter besonderen Sicherheitsmaßnahmen handhabbar. Darüberhinaus gelingt die Alkylierung nur mit einer Enantioselektivität von 3,5:1, d.h. es werden etwa 25 % des (2S,3R)-Enantiomers gebildet.

Auch die Anwendung des teuren Trifluormethansulfonsäureethylester als erforderliches Alkylierungsmittel steht einer wirtschaftlich lohnenden Anwendung im Wege.

Nach D. Seebach et al., Angew. Chemie 93 (1981) Nr. 11, S. 1007-1008, kann man den di-tert.-Butylester der β-Ethylasparaginsäure auch durch Umsetzung von N-Formyl Asparaginsäure di-tert.-Butylester mit Lithiumdiethylamid und Ethyljodid erhalten. Jedoch entsteht hierbei auch das in α-Stellung alkylierte Produkt.

Nach M. Akhtar et al., Tetrahedron 43, 5899-5908 (1987) läßt sich (2S,3S)-3-Ethylasparaginsäure aus 2-Ethylfumarsäure durch Umsetzung mit Ammoniak in Gegenwart von 3-Methylaspartase aus Clostridium tetanomorphum nach Schema 2 herstellen.

Schema 2

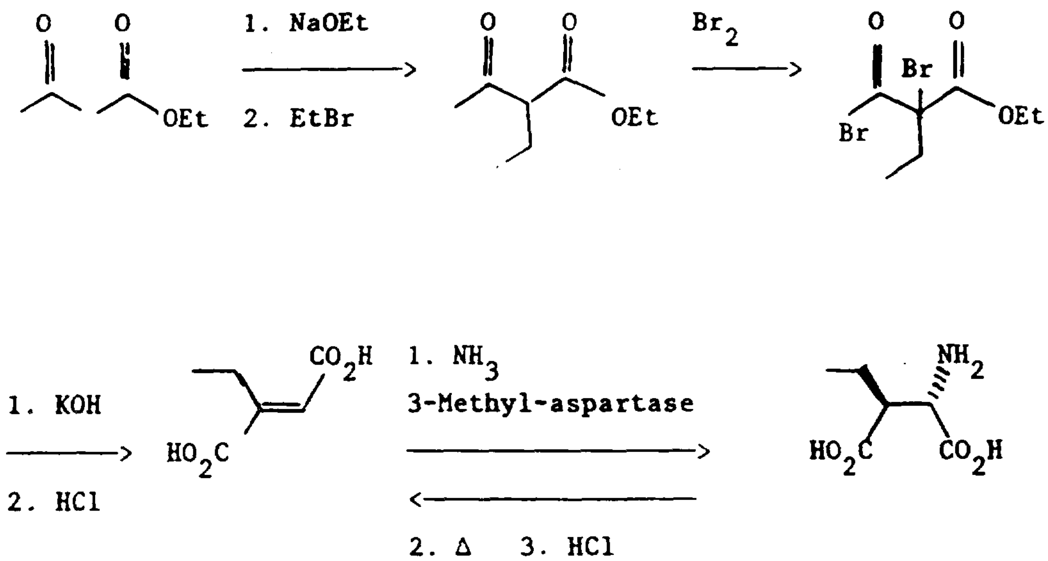


Aufgabe der vorliegenden Erfindung war es nun ein Verfahren zur Herstellung von N-geschützten (2S,3S)-3-Ethylasparaginsäuredialkylestern bereitzustellen, das die Nachteile der bekannten Verfahren nicht oder lediglich in geringem Umfang aufweist.

Überraschenderweise wurde gefunden, daß die Umsetzung von durch enzymatische Aminierung erhältlicher (2S, 3S)-Ethylasparaginsäure, gemäß Schema 3, mit einem Halogenierungsmittel in Gegenwart eines Alkanols zu (2S,3S)-3-Ethylasparaginsäuredialkylester führt.

Schema 3

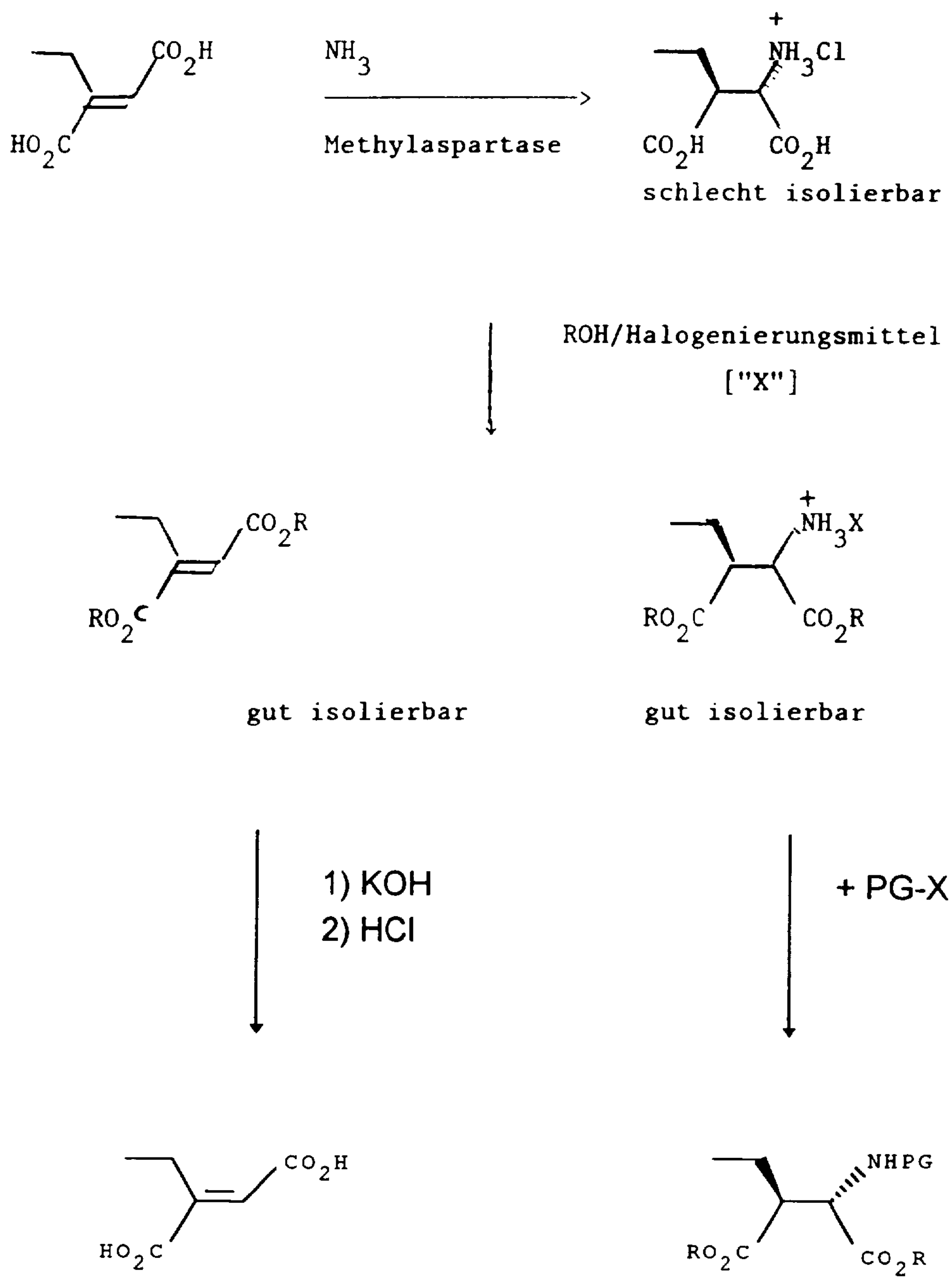


PG bedeutet eine geeignete Aminoschutzgruppe
X Bedeutet eine Abgangsgruppe

Gegenstand der Erfindung ist somit ein Verfahren zur Herstellung von N-geschützten (2S,3S)-3-Ethylasparaginsäuredialkylestern aus ungereinigter, durch enzymatische Aminierung von Ethylfumarsäure erhältlicher (2S,3S)-3-Ethylasparaginsäure, wobei man das Produktgemisch der enzymatischen Aminierung in Gegenwart eines Alkanols mit einem Halogenierungsmittel behandelt, den erhaltenen 2-Ethylfumarsäuredialkylester abtrennt, und die freie Aminogruppe mit einer Schutzgruppe versieht, insbesondere ein Verfahren, wobei man das Produktgemisch mit Thionylchlorid in Gegenwart von Methanol oder Ethanol behandelt.

Die Durchführung des erfindungsgemäßen Verfahrens gestaltet sich einfach. Das durch die enzymatische Aminierung der 3-Ethylfumarsäure gewonnene ungereinigte Produktgemisch wird in dem Alkanol aufgenommen und mit

dem Halogenierungsmittel versetzt. Als Alkanol werden vorzugsweise unverzweigte oder verzweigte Alkohole mit 1 bis 6 C-Atomen, wie z.B. Methanol, Ethanol, n-Propanol, Isopropanol oder n-Butanol, insbesondere Methanol oder Ethanol verwendet. Bevorzugte Halogenierungsmittel sind Thionylchlorid, Phosphortrichlorid, Phosphorpentachlorid, Phosphortribromid, Phosphorpentabromid, Antimontrichlorid, Antimonpentachlorid oder Triphenylphosphin in Tetrachlormethan, insbesondere Thionylchlorid.

In der Regel werden 10 g des durch enzymatische Aminierung erhaltenen ungereingten Produktgemisches in 50 ml bis 250 ml, vorzugsweise 100 ml bis 200 ml, des betreffenden Alkanols suspendiert und mit 5 bis 30 ml, vorzugsweise 10 bis 25 ml des einzusetzenden Halogenierungsmittel versetzt. Die Zugabe erfolgt zwischen -20° und +20 °C, vorzugsweise zwischen -10° und +10 °C, insbesondere um den Gefrierpunkt von Wasser.

In der Regel wird die Reaktion bei Temperaturen zwischen 0 und 80 °C, vorzugsweise 20° und 60 °C, insbesondere bei Raumtemperatur, mit einer Reaktionszeit von 15 bis 96 Stunden, vorzugsweise 48 bis 72 Stunden, durchgeführt.

Zur Aufarbeitung der Reaktionsmischung wird diese, vorzugsweise unter reduziertenm Druck, eingeengt und der so erhaltene Rückstand mit einem unpolaren bis leicht polaren Lösungsmittel, vorzugsweise mit einem Kohlenwasserstoff, wie z.B. n-Pentan, n-Hexan, Cyclohexan oder Toluol oder mit einem Ether, wie z.B. Diethylether, Tetrahydrofuran, Dioxan, Methyl-tertiär-butylether, extrahiert. Nach Eindampfen des so erhaltenen Filtrats gewinnt man die bei der enzymatischen Aminierung nicht umgesetzte 3-Ethylfumarsäure als Alkylester vollständig zurück. Diese kann nach Verseifung wieder bei der enzymatischen Aminierung eingesetzt werden.

Der Filterrückstand wird mit einem polaren Lösungsmittel, vorzugsweise mit Ketonen, wie z.B. Aceton, Cyclohexanon oder Methylethylketon, Amiden wie z.B. Dimethylformamid, vorzugsweise durch Auskochen extrahiert und durch Umkristallisation gereinigt. Das so erhaltene Rohprodukt kann jedoch auch ohne vorherige Reinigung zur weiteren Umsetzung zu einen γ-Lactam verbrückten 2S,3R,2'S-Isoleucin-Alanin-Dipeptids verwendet werden.

PG bedeutet vorzugsweise eine Schutzgruppe für das mit ihr verknüpfte Stickstoffatom, die es ermöglicht, die Verbindung der Formel I in ein γ-Lactam-verbrücktes 2S,3R-2'S-Isoleucin-Alanin-Dipeptid, dessen Stickstoffatom mit PG geschützt ist, mit dem z.B. von H. Rapoport und J.P. Wolf (doc. cit.) beschriebenen Verfahren überzuführen, und anschließend diese Schutzgruppe unter milden Bedingungen selektiv abzuspalten.

Solche Schutzgruppen sind dem Fachmann geläufig (z.B. Protective Groups in Organic Chemistry, Plenum Press, New York, 1973) und können nach bekannten Methoden eingeführt und abgespalten werden. Bevorzugte Schutzgruppe PG sind beispielsweise Benzyl, Methoxybenzyl, 3,4-Dimethoxybenzyl, 4-Methylbenzyl, Allyl, Methally, Crotyl, Trimethylsilyl oder tert.-Butyldimethylsilyl, Diphenylmethyl, Trityl, 9-H-Fluorenyl, 9-Phenyl-9-Fluorenyl, Methoxymethyl oder Formyl.

Die Einführung der Formylgruppe kann z.B. nach L.I. Krimen, Org. Synth. 50, 1 (1970) durchgeführt werden.

N-geschützte (2S,3S)-3-Ethylasparaginsäuredialkylester stellen wertvolle Zwischenprodukte bei der Synthese von γ-Lactam-verbrücktem 2S,3R,2'S-Isoleucin-Alanin-Dipeptid dar. Sie können leicht und in hohen Ausbeuten durch Behandeln des Produktgemisches der enzymatischen Aminierung von Ethylfumarsäure mit einem Halogenierungsmittel in Gegenwart eines Alkanols erhalten werden.

Die nachfolgenden Ausführungsbeispiele sind zum Zweck der Illustration angegeben und sollen nicht beschränkend sein. Das erfindungsgemäße Verfahren kann in vielen Variationen durchgeführt werden, so können z.B. andere organische Lösungsmittel verwendet werden und die Reaktionstemperaturen und Reaktionszeiten variiert werden.

Herstellung der Ausgangsstoffe:

1. Ethylfumarsäure

Ethylfumarsäure wurde ausgehend von Acetessigester durch Ethylierung, Bromierung und anschließender KOH-Behandlung in 40 % Ausbeute erhalten [D. Gani et al., Tetrahedron 24, 5904 (1987)].

2. Kultur und Ernte der Zellen

Zur Gewinnung von Zellen von Clostridium spec., DSM 528, erfolgte die Kultivierung in 1000 ml Enghals-Erlenmeyerkolben in folgender Nährlösung:

| | |
|---|---|
| $K_2HPO_4$ | 2,9 g |
| $KH_2PO_4$ | 2,3 g |
| $MgSO_4 . 7 H_2O$ | 0,025 g |
| $CaCl_2 . 2 H_2O$ | 0,015 g |
| $FeSO_4 . 7 H_2O$ | 0,01 g |
| $MnCl_2 . 4 H_2O$ | 0,002 g |

Fortsetzung der Tabelle auf der nächsten Seite

(fortgesetzt)

| | |
|---|---|
| $CoCl_2 . 6H_2O$ | 0,0025 g |
| $Na_2MoO_4 . 2 H_2O$ | 0,0025 g |
| Na-Glutamat | 17 g |
| Hefeextrakt | 6 g |
| Na-Thioglykolat | 0,5 g |
| Aqua dest. ad 1000 ml, pH-Wert: 7,1. | |

Das Medium wurde 20 min bei 121 °C autoklaviert.

Inokuliert wurden pro Kolben ca. 5 ml einer gut gewachsenen Übernacht-Kultur. Die Kulturen wurden bei 37 °C unter ständigem schwachen Schütteln für 40-64 Stunden bebrütet. Nach Abschluß des Wachstums wurden die Zellen durch Zentrifugation bei 6000 g sedimentiert, zweimal mit kalter 0,9%iger NaCl gewaschen. Die Zellausbeute betrug durchschnittlich 4 g Zellen/l Kulturmedium. Für Umsetzungsversuche wurden die Zellen entweder sofort eingesetzt oder bei -18 °C eingefroren.

3. Gewinnung von zellfreien Extrakten

Zur Gewinnung zellfreier Extrakte wurden die gewaschenen Zellen (bzw. eingefroreren Zellen) pro Gramm Naßgewicht mit 1 ml 0,01 M $KH_2PO_4/K_2HPO_4$-Puffer, pH-Wert 7,4 und 2 g Glasperlen (Ø 0,01 ml) versetzt und im Zellhomogenisator MSK der Fa. Braun, Melsungen, bei 4000 U/min unter ständiger $CO_2$-Kühlung aufgeschlossen. Glasperlen und Zelltrümmer wurden durch Zentrifugation bei 6000 g in 30 min abgetrennt. Der zellfreie Extrakt wurde entweder ohne weitere Behandlung oder nach Teilreinigung der β-Methylaspartat-Ammonia-Lyase zur Aminierung von Ethylfumarat eingesetzt.

4. Teilreinigung der β-Methyl-Aspartat-Ammonia-Lyase

Protaminsulfat-Fällung

Der Rohextrakt wurde tropfenweise mit einer frisch bereiteten 1%igen Protaminsulfat-Lösung (1 ml pro 100 mg Protein) bei 0 °C versetzt. Die Lösung wurde 10 min nachgerührt, der Niederschlag sedimentiert und verworfen.

Ammoniumsulfat-Fällung

Der Überstand der Protaminsulfat-Fällung wurde pro 10 ml mit 2 ml 1 M Phosphatpuffer, pH-Wert 7,4 versetzt; anschließend wurde durch Zugabe von gesättigter Ammoniumsulfat-Lösung eine 50%ige Sättigung eingestellt und 10 min nachgerührt. Ausgefallenes Protein wurde abzentrifugiert und verworfen. Anschließend wurde die Sättigung an $(NH_4)_2SO_4$ auf 60 % erhöht und ebenfalls 10 min nachgerührt. Gefälltes Protein wurde abzentrifugiert, der Niederschlag in wenig Phosphatpuffer resuspendiert und für die enzymatische Aminierung eingesetzt.

5. Enzymatische Aminierung von 3-Ethyl-fumarsäure

Rohextrakte oder teilgereinigte Enzympräparate wurden zur enzymatischen Aminierung von Ethylfumarat eingesetzt.

Beispiel

| | |
|---|---|
| 480 ml | Tris/HCl-Puffer, 0,1 M, pH-Wert 8,4 |
| 24 ml | KCl, 0,1 M |
| 24 ml | $MgCl_2 . 6 H_2O$ |
| 72 ml | $NH_3$ (5%ig) |
| 2,2 ml | Ethylfumarat (nach 1.) |
| 2,5 ml | Enzymlösung (nach 4.) |

Reaktionsdauer: 30 Minuten bei 30 °C. Abbruch der Reaktion durch Hitzebehandlung des Ansatzes (100 °C, 10 Minuten). Danaturiertes Protein wurde sedimentiert und verworfen. Der klare Überstand wurde gefriergetrocknet. Die Rohausbeute betrug 10,3 g

Beispiel 1

(2S,3S)-3-Ethylasparaginsäuredimethylesthydrochlorid

Das gefriergetrocknete Produkt (10,3 g) der enzymatischen Aminierung wurde in 160 ml Methanol weitgehend

gelöst und bei 0 °C mit 15 ml Thionylchlorid langsam versetzt. Die dabei entstehende Suspension wurde 3 Tage bei Raumtemperatur gerührt und anschließend im Vakuum eingeengt. Der feste Rückstand (14 g) wurde mit 200 ml Methyl-tertiär-butylether 2,5 h ausgekocht und über einen Filtertiegel abgesaugt. Das Filtrat wurde im Vakuum eingeengt. Zurück blieben 1,2 g (7,0 mmol, 46 %) 3-Ethylfumarsäuredimethylester als leicht braunes Öl.

Der Filterrückstand wurde mit 200 ml Aceton 2 h ausgekocht und heiß abfiltriert. Das Filtrat wurde im Vakuum eingeengt. Zurück blieben 1,8 g (7,9 mmol; 52 %) (2S,3S)-3-Ethylasparaginsäuredimethylesterhydrochlorid als beiges Pulver: $[\alpha]_D^{25}$= +18,3° (c 0,1 in $CHCl_3$); $^1$H-NMR (200 MHz, $CDCl_3$): $\delta$ = 1,05 (t; 3H, $CH_3$); 1,80-2,15 (m; 2H, $CH_2$); 3,25 (m; 1H, 3-H); 3,20 und 3,85 (2s; je 3 H, $OCH_3$); 4,45 (breites d, 1H, 2-H); 8,80 (breites s; 3H, $NH_3^+$). $^{13}$C-NMR (50,32 MHz, $CDCl_3$): $\delta$ = 11,96 (q; $CH_3$); 22,43 (t; $CH_2$); 47,36 (d; C-3); 52,64 und 53,42 (2q; $OCH_3$); 55,07 (d; C-2); 166,37 und 172,30 (2s; C-1 und C4).

Unter Verwendung von Ethanol erhält man analog (2S,3S)-3-Ethylasparaginsäurediethylesterhydrochlorid.

Beispiel 2

Zurückgewinnung von 2-Ethylfumarsäure

1,2 g (7,0 mmol) 2-Ethylfumarsäuredimethylester aus Beispiel 1 wurden mit 20 ml 1N NaOH-Lösung versetzt und 2 h am Rückfluß gekocht. Die erkaltete Lösung wurde mit konzentrierter Salzsäure auf pH 1 gebracht und mit Diethylether extrahiert. Die organische Phase wurde über $MgSO_4$ getrocknet und anschließend im Vakuum eingeengt. Zurück blieben 0,95 g (6,6 mmol, 94 %) 2-Ethylfumarsäure vom Erweichungspunkt 180 °C. Die so gewonnene 2-Ethylfumarsäure konnte problemlos - wie unter 5. beschrieben - wieder eingesetzt werden.

Beispiel 3

(2S,3S)-3-Ethylasparaginsäure

1,0 g (4,4 mmol) (2S,3S)-3-Ethylasparaginsäuredimethylesterhydrochlorid aus Beispiel 1 wurden in 50 ml konzentrierter Salzsäure 2 Tage am Rückfluß gekocht. Der Ansatz wurde im Vakuum eingeengt. Der Rückstand wurde in 7 ml Wasser gelöst, mit 1N NaOH auf pH 3 gebracht und mit 150 ml Ethanol verdünnt. Nach 2 Tagen im Kühlschrank waren 0,5 g (3,5 mmol; 80 %) (2S,3S)-3-Ethylasparaginsäure vom Schmelzpunkt 239-241 °C ausgefallen. $[\alpha]_D^{25}$= +19° (c 0,1 in 1N HCl).

$^1$H-NMR (250 MHz, $D_2O$): $\delta$ = 1,00 (t; 3H, $CH_3$); 1,60 und 1,75 (2m; je 1H, $CH_2$ ); 2,90 (m; 1H, H-3); 4,05 (d; 1H, H-2). MS (70eV); m/e = 116 ($M^+$-$CO_2$H; 53 %); 56 (100).

Beispiel 4

(2S,3S)-2-(N-Phenylfluorenyl)-3-ethylasparaginsäuredimethylester

Zu einer Suspension von 2,5 mmol $Pb(NO_3)_2$ und 6,0 mmol $K_3PO_4$ in 15 ml Acetontril werden nacheinander 3,0 mmol des nach Beispiel 3 hergestellten Produkts und 3,5 mmol Phenylfluorenylbromid gegeben.

Nach 30stündigem Rühren bei Raumtemperatur wird die Reaktionsmischung gefiltert und der Filterrückstand mit 40 ml Dichlormethan gewaschen. Die vereinten Filtrate werden eingeengt. Der Rückstand wird chromatographiert (Kieselgel, Laufmittel: Essigester-Hexan: 1:5) und man erhält das reine Produkt, dessen 1H NMR-Daten mit denen in J. Org. Chem., Vol. 54, No. 13, 1989, S. 3172 identisch sind.

Beispiel 5

(2S,3S)-2-(N-Benzyl)-3-ethylasparaginsäuredimethylester

Zu einer Suspension von 3,0 mmol des nach Beispiel 3 hergestellten Produkts und 3,1 mmol Benzaldehyd in 25 ml Methanol werden 3,3 mmol Natriumcyanoborhydrid gegeben. Nach 1stündigem Rühren wird die Reaktionsmischung wie üblich aufgearbeitet. Der Rückstand wird chromatographiert (Kieselgel, Laufmittel: Essigsäure-Hexan: 1: 4), und man erhält das reine Produkt:

H-NMR (250 MHz, $CDCl_3$): $\delta$ = 0,5 (t, 3H, $CH_3$), 0,9-1,1 (m, 2H, $CH_2$), 2,5 (m, 1H, CCH), 2,9 (s, 3H, $OCH_3$), 3,6 (s, 3H, $OCH_3$), 3,8 (d, 1H, N-CH), 4,3-4,6 (m, 2H, Ph-$CH_2$), 7,3-7,35 (m, 5H, Ph-H).

Vergleichsbeispiel

2,5 g (5,8 mmol) N-Phenylfluorenyl-(2S,3S)-3-Ethylasparaginsäuredimethylester, hergestellt nach J. Org. Chem. 54, 3164-3173 (1989), wurden mit 50 ml konzentrierter Salzsäure versetzt und 2 Tage unter Rückfluß erhitzt. Der erkaltete Ansatz wurde zweimal mit je 25 ml Toluol gewaschen. Die organische Phase wurde verworfen und die wäßrige Phase im Vakuum eingeengt. Der Rückstand wurde in 5 ml $H_2O$ gelöst mit 1N NaOH auf pH 3 gebracht und mit 100 ml Ethanol überschichtet. Nach 20 h bei 0 °C fielen 0,4 g (2,8 mmol, 48 %) (2S,3S)-3-Ethylasparaginsäure vom Schmelzpunkt 252-253 °C aus. $[\alpha]_D^{25}$ = +18° (c 0,1 in 1N HCl); spektroskopische Daten sind identisch mit denen der nach Beispiel 3 erhaltenen Verbindung.

## Patentansprüche

1. Verfahren zur Herstellung von N-geschützten (2S,3S)-3-Ethylasparaginsäuredialkylestern aus ungereinigter, durch enzymatische Aminierung von Ethylfumarsäure erhältlicher (2S,3S)-3-Ethylasparaginsäure, dadurch gekennzeichnet, daß man

   - das Produktgemisch der enzymatischen Aminierung in Gegenwart eines Alkanols mit einem Halogenierungsmittel behandelt,
   - den erhaltenen 2-Ethylfumarsäuredialkylester abtrennt, und
   - die Aminogruppe mit einer Schutzgruppe versieht.

2. Verfahren nach Anspruch 1, dadurch gekennzeichnet, daß man das Produktgemisch mit Thionylchlorid behandelt.

3. Verfahren nach Anspruch 1 oder 2, dadurch gekennzeichnet, daß man das Produktgemisch in Gegenwart von Methanol oder Ethanol behandelt.

## Claims

1. Process for the preparation of N-protected dialkyl (2S,3S)-3-ethylaspartates from unpurified (2S,3S)-3-ethylaspartic acid obtainable by enzymatic amination of ethylfumaric acid, characterized in that

   - the product mixture from the enzymatic amination is treated with a halogenating agent in the presence of an alkanol,
   - the dialkyl 2-ethylfumarate obtained is separated off, and
   - the amino group is provided with a protective group.

2. Process according to Claim 1, characterized in that the product mixture is treated with thionyl chloride.

3. Process according to Claim 1 or 2, characterized in that the product mixture is treated in the presence of methanol or ethanol.

## Revendications

1. Procédé de préparation d'esters dialkyliques de l'acide (2S,3S)-3-éthylaspartique N-protégés à partir d'acide (2S, 3S)-3-éthylaspartique non purifié, obtenu par amination enzymatique d'acide éthylfumarique, caractérisé en ce que

   - on traite le mélange produit de l'amination enzymatique en présence d'un alcanol avec un agent halogénant,
   - on sépare l'ester dialkylique de l'acide 2-éthylfumarique obtenu, et
   - on munit le groupe amino d'un groupe protecteur.

2. Procédé selon la revendication 1, caractérisé en ce qu'on traite le mélange produit avec du chlorure de thionyle.

3. Procédé selon la revendication 1 ou 2, caractérisé en ce qu'on traite le mélange produit en présence de méthanol

ou d'éthanol.